(19) Europäisches Patentamt
European Patent Office
Office européen des brevets

(11) Numéro de publication: **0 077 427**
**B1**

(12) # FASCICULE DE BREVET EUROPEEN

(45) Date de publication du fascicule du brevet: **22.05.85**

(51) Int. Cl.⁴: **C 07 D 211/46**, A 61 K 31/445

(21) Numéro de dépôt: **81401604.4**

(22) Date de dépôt: **15.10.81**

(54) **Dérivés de pipéridine, leur procédé de préparation et leur application en thérapeutique.**

(43) Date de publication de la demande:
**27.04.83 Bulletin 83/17**

(45) Mention de la délivrance du brevet:
**22.05.85 Bulletin 85/21**

(84) Etats contractants désignés:
**AT BE CH DE FR GB IT LI LU NL SE**

(56) Documents cités:
**EP - A - 0 015 817**
**DE - A - 1 964 515**
**FR - A - 2 481 278**
**GB - A - 2 056 447**

(73) Titulaire: **SYNTHELABO, 58, rue de la Glacière, F-75621 Paris Cedex 13 (FR)**

(72) Inventeur: **Schneider, Roland, 88, rue Pasteur, F-78700 Conflans-Ste-Honorine (FR)**
Inventeur: **Warolin, Christian, 55, Boulevard Beauséjour, F-75016 Paris (FR)**
Inventeur: **Bigg, Dennis, 5, Parc de Diane, F-78350 Jouy-en-Josas (FR)**

(74) Mandataire: **Thouret-Lemaitre, Elisabeth, Service Brevets - SYNTHELABO 58, rue de la Glacière, F-75621 Paris Cedex 13 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

La présente invention concerne des dérivés de pipéridine, leur procédé de préparation et leur application en thérapeutique.

Les composés de l'invention répondent à la formule (I)

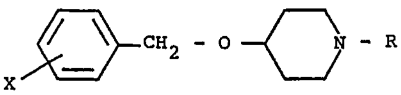

dans laquelle:
R représente soit un atome d'hydrogène, soit un radical ($C_{1-4}$) alkyle, soit un radical hydroxy-($C_{1-4}$) alkyle, soit un radical ($C_{1-4}$) alcoxycarbonyle, soit un radical benzyle pouvant porter un substituant choisi parmi les atomes d'halogène et les radicaux ($C_{1-4}$) alcoxy, soit le radical phénéthyle, soit le radical phényl-3 propyle,
X représente un ou plusieurs atomes d'hydrogène ou d'halogène ou radicaux ($C_{1-4}$) alkyle, ($C_{1-4}$) alcoxy, trifluorométhyle, méthylènedioxy, ou bien X forme avec le noyau phényle un radical naphtyle, R et X ne pouvant représenter simultanément des atomes d'hydrogène.

Les sels d'addition que forment les composés (I) avec les acides pharmaceutiquement acceptables font partie de l'invention.

Le composé de formule I dans laquelle R et X représenteraient chacun un atome d'hydrogène est décrit dans la demande de brevet européen No 15817.

Ce composé connu est un antispasmodique; mais, contrairement aux composés de la présente invention, il est inactif comme antidépresseur.

Les composés préférés de l'invention sont ceux dans lesquels X représente un ou plusieurs atomes de chlore, ou forme avec le noyau phényle un radical naphtyle ou encore représente trois radicaux méthoxy.

Parmi ces composés sont à considérer plus particulièrement ceux dans lesquels R est H.

Selon l'invention, on peut préparer les composés (I) à partir d'un composé (II)

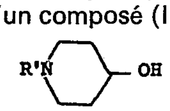

dans lequel R' représente un radical protecteur de l'azote tel que le radical nitro-4 benzoyle, benzoyle éventuellement substitué, benzyle éventuellement substitué ou alkyle, que l'on fait réagir avec un composé (III)

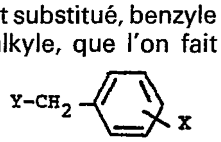

dans lequel Y représente un radical réactif tel qu'un atome de chlore ou de brome; puis soit on élimine le radical protecteur R' s'il ne correspond pas à un radical R, soit on réduit le radical protecteur benzoyle éventuellement substitué, soit on fait réagir un composé (I)

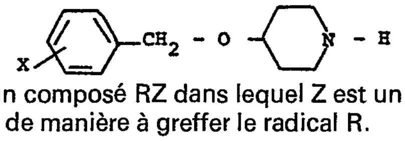 (I)

avec un composé RZ dans lequel Z est un groupe réactif de manière à greffer le radical R.

Les exemples suivants illustrent l'invention. Les analyses et les spectres IR et RMN confirment la structure des composés.

*Exemple 1: (Fluoro-4 benzyloxy-4 pipéridine et son mandélate*

1.1 *(Nitro-4 benzoyl)-1 (fluoro-4 benzyloxy)-4 pipéridine*

Un mélange de 1,6 g de soude aqueuse à 50%, de 10 ml de dichlorométhane, de 2 g (0,008 mol) de (nitro-4-benzoyl)-1 hydroxy-4 pipéridine, de 2,32 g (0,012 mol) de bromure de fluoro-4 benzyle et de 0,15 g ($4 \times 10^{-4}$ mol) d'iodure de tétrabutylamonnium est agité 5 h à 50° C. Après dilution du milieu réactionnel par 20 ml d'eau et 30 ml de dichlorométhane, la phase organique est décantée, lavée à l'eau jusqu'à neutralité, séchée ($MgSO_4$) et évaporée sous vide. L'huile résiduelle obtenue cristallise par agitation dans l'éther.

On obtient 2,23 g d'un produit qui est purifié par dissolution dans 11 ml de diméthylformamide (DMF) et reprécipitation par addition goutte à goutte de 11 ml d'eau, sous agitation. Après lavage à l'eau et séchage sous vide, on obtient la (nitro-4 benzoyl)-1 (fluoro-4 benzyloxy)-4 pipéridine. P.f. = 120° C.

1.2. *(Fluoro-4 benzyloxy)-4 pipéridine (mandélate)*

Un mélange de 11,48 g (0,032 mol) du produit obtenu en 1.1, de 192 ml d'éthanol à 96° et de 48 ml de potasse aqueuse 10M est agité sous azote et porté 4 h à 50° C. L'alcool est chassé sous vide, le résidu repris par 200 ml d'eau, 72 g de chlorure de sodium et 200 ml d'éther. Après filtration, la phase aqueuse est extraite deux fois par 200 ml d'éther, la phase organique totale est séchée ($MgSO_4$) et évaporée sous vide.

On obtient une huile qui est transformée en d,l-mandélate par dissolution dans 100 ml d'éther et adjonction de 4,57 g (0,03 mol) d'acide d,l-mandélique. Le sel obtenu est filtré et recristallisé dans de l'isopropanol. P.f. = 146° C.

*Exemple 2: (Triméthoxy-3,4,5 benzyloxy)-4 pipéridine et son mandélate*

2.1. *(Nitro-4 benzoyl)-1 (triméthoxy-3,4,5 benzyloxy)-4 pipéridine*

Un mélange de 0,8 g de soude aqueuse à 50%, de 5 ml de dichlorométhane, de 1 g (0,004 mol) de (nitro-4 benzoyl)-1 hydroxy-4 pipéridine, de 1,30 g (0,006 mol) de chlorure de triméthoxy-3,4,5 benzyle et de 0,08 g ($2 \times 10^{-4}$ mol) d'iodure de tétrabutylammonium est agité 5 h à 50° C. Après dilution par 10 ml d'eau et 15 ml de dichlorométhane, la phase organique est décantée, lavée à l'eau à neutralité, séchée ($MgSO_4$) et évaporée sous vide. L'huile résiduelle obtenue cristrallise par agitation dans l'éther.

On obtient 1,36 g (79%) d'un produit qui est purifié par dissolution dans 7 ml de DMF et reprécipitation lente par 7 ml d'eau. Après lavage à l'eau et séchage sous vide, on obtient la (nitro-4 benzoyl)-1 (triméthoxy-3,4,5 benzyloxy)-4 pipéridine. P.f. = 124° C.

2.2 *(Triméthoxy-3,4,5 benzyloxy)-4 pipéridine (mandélate)*

Un mélange de 12 g (0,028 mol) du produit obtenu en 2.1, de 168 ml d'éthanol à 96° et de 48 ml de potasse aqueuse 10M est agité sous azote et porté 4 h à 50° C. L'alcool est chassé sous vide, le résidu repris par 70 ml d'eau, 25,2 g de chlorure de sodium et 70 ml de chloroforme. Après filtration, la phase aqueuse est extraite par deux fois 70 ml de chloroforme, la phase organique totale est séchée ($MgSO_4$) et évaporée sous vide.

On obtient un solide pâteux qui est transformé en d,l-mandélate par dissolution dans 120 ml de méthanol, adjonction de 4,26 g (0,028 mol) d'acide d,l- mandolique et filtration après agitation du milieu pendant une nuit à la température ambiante. Le précipité obtenu est recristallisé dans du méthanol. Le produit cristallise avec une demi-molécule d'eau. P.f. = 114° C.

*Exemple 3: Ethoxycarbonyl-1 benzyloxy-4 pipéridine*

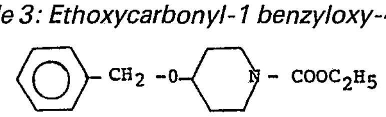

A un mélange agité de 6,83 g (0,03 mol) de chlorhydrate de benzyloxy-4 pipéridine, de 7,74 g (0,056 mol) de $K_2CO_3$ sec, de 26 ml d'eau et de 26 ml de chloroforme, on ajoute goutte à goutte 3,4 g (0,033 mol) de chloroformiate d'éthyle. Après 1 h d'agitation, la phase organique est décantée après dilution avec 54 ml de chloroforme, lavée avec trois fois 20 ml d'eau et séchée ($MgSO_4$). L'évaporation du solvant sous vide permet d'obtenir un liquide qui est distillé sous vide. $Eb_{0,4}$: 149-151° C; $n_D^{21}$ = 1,5178

*Exemple 4: Méthyl-1 (chloro-4 benzyloxy)-4 pipéridine et son chlorydrate*

Dans un ballon tricol de 500 ml placé sous atmosphère d'argon, on lave trois fois à l'éther de pétrole 4,4 g (0,1 mol) d'hydrure de sodium à 55% dans de l'huile. On ajoute ensuite 10,1 g (0,1 mol) de méthyl-1 hydroxy-4 pipéridine en solution dans 100 ml de DMF. On agite 1 h à la température ambiante puis on refroidit par un bain d'eau glacée et on ajoute 19,3 g (0,12 mol) de chlorure de p-chlorobenzyle en solution dans 50 ml de DMF. Une fois l'introduction terminée, on agite 4 h à la température ambiante et laisse au repos une nuit. On verse le milieu réactionnel dans de l'eau glacée et extrait à l'éther trois fois. La phase organique est lavée une fois à l'eau puis on extrait avec HCl dilué (1-2N). La phase aqueuse est ensuite alcalinisée par NaOH. On extrait à l'éther puis lave à l'eau quatre fois. On sèche sur sulfate de magnésium, filtre et concentre. L'huile obtenue est distillée deux fois et on recueille la fraction passant à 94-98° C/ 0,04 mmHg.

On obtient une huile que l'on reprend dans de l'éther et on fait précipiter le chlorhydrate en ajoutant du gaz chlorhydrique. On filtre et rince à l'éther le chlorhydrate. On le reprend dans le minimun d'isopropanol à chaud puis on ajoute cinq fois le volume d'acétate d'éthyle et on laisse recristalliser le composé. P.f. = 149-151° C.

*Exemple 5: Benzyl-1 (triméthoxy-3,4,5 benzyloxy)-4 pipéridine et son fumarate*

Dans un ballon tricol de 250 ml placé sous atmosphère d'azote, on lave trois fois à l'éther de pétrole 2,2 g (0,05 mol) d'hydrure de sodium à 55% dans de l'huile. On introduit ensuite 9,6 g (0,05 mol) de benzyl-1 hydroxy-4 pipéridine en solution dans 30 ml de DMF. Une fois l'addition terminée, on agite 1 h à la température ambiante. On ajoute ensuite, en refroidissant par un bain d'eau glacée, 13 g (0,06 mol) de chlorure de triméthoxy-3,4,5 benzyle dans 30 ml de DMF. On agite 5 h à la température ambiante puis on laisse reposer une nuit. On verse le milieu réactionnel dans de l'eau glacée puis extrait à l'éther. Le produit est ensuite extrait avec HCl dilué. La phase aqueuse est alcalinisée par NaOH puis on extrait à l'éther, on lave à l'eau, sèche sur $MgSO_4$, filtre et concentre. L'huile obtenue est reprise dans du pentane à chaud. Le produit cristallise. On le filtre et le recristallise dans de l'isopropanol.

On obtient la base que l'on dissout dans 70 ml d'éthanol et ajoute une solution filtrée de 2,9 g (0,025 mol) d'acide fumarique dans 140 ml d'éthanol. On filtre et sèche le sel formé. P.f. = 160-161° C.

*Exemple 6: (Chloro-4 benzyl)-1 (triméthoxy-3,4,5 benzyloxy)-4 pipéridine et son fumarate*

6.1 *(Chloro-4 benzoyl)-1 hydroxy-4 pipéridine*

Dans un erlenmeyer de 1 l, on place 30 g (0,296 mol) d'hydroxy-4 pipéridine, 260 ml de $CHCl_3$, 57,3 g (0,414 mol) de $K_2CO_3$ et 260 ml d'eau. On ajoute en 15 min, en refroidissant par un bain d'eau glacée, 51,8 g (0,296 mol) de chlorure de p-chlorobenzoyle dissous dans 50 ml de $CHCl_3$. On agite une nuit à la température ambiante. On décante, extrait avec $CHCl_3$ et lave à l'eau jusqu'à pH 6-7. On sèche sur $MgSO_4$, filtre et concentre. On recristallise le composé dans de l'acétate d'éthyle.

6.2 *(Chloro-4 benzoyl)-1 (triméthoxy-3,4,5 benzyloxy)4 pipéridine*

Dans un ballon tricol, sous atmosphère d'azote, on lave trois fois à l'éther de pétrole 0,96 g (0,022 mol) de NaH à 55% dans de l'huile. On ajoute ensuite 4,8 g (0,02 mol) du composé obtenu sous 6.1 dissous dans 50 ml de DMF. On agite 1 h après la fin de l'addition à la température ambiante. On refroidit ensuite par un bain d'eau glacée et on ajoute 5,4 g (0,025 mol) de chlorure de triméthoxy-3,4,5 benzyle dissous dans 10 ml de DMF. On agite 3 h à la température ambiante, puis on laisse au repos une nuit. On verse dans de l'eau glacée et extrait avec de l'acétate d'éthyle. On lave à l'eau, sèche sur $MgSO_4$, filtre et concentre. L'huile obtenue est reprise dans de l'éther puis filtrée. Le filtrat est concentré et passé sur colonne d'alumine (éluant $CHCl_3$). Le produit ne cristallise pas et est utilisé tel quel dans l'étape suivante.

6.3 *(Chloro-4 benzyl)-1 (triméthoxy-3,4,5 benzyloxy)-4 pipéridine et son fumarate*

Dans un ballon, sous atmosphère d'azote, on ajoute à la température ambiante, à une suspension de 0,38 g (0,01 mol) de $AlLiH_4$ dans 30 ml d'éther sec, 7 g (0,0167 mol) du composé obtenu sous 6.2, dissous dans 70 ml d'éther sec. On chauffe 3 h à la température du reflux. On hydrolyse par 2,6 ml d'isopropanol et 3,3 ml d'eau saturée par NaCl. On filtre et rince à l'éther. On extrait le produit dans de l'acide chlorhydrique dilué puis on alcalinise le mélange réactionnel avec $NH_4OH$ et extrait à l'éther. On lave à l'eau, sèche sur $MgSO_4$, filtre et concentre.

On obtient une huile que l'on dissout dans 30 ml d'éthanol et ajoute à une solution filtrée de 1,2 g (0,0105 mol) d'acide fumarique dans 60 ml d'éthanol. Le fumarate précipite doucement. On le filtre, le rince avec un peu d'éthanol puis à l'éther et le sèche. P.f. = 178-180° C.

Les composés préparés à titre d'exemples sont représentés dans le tableau.

*Tableau*

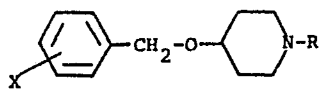 (I)

| Composé | X | R | Sel | P.f. (° C) |
|---|---|---|---|---|
| 1 | 4-F | H | d,l-mandélate | 146 |
| 2 | 3,4(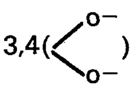) | H | d,l-mandélate | 137 |
| 3 | 3,4,5-$(OMe)_3$ | H | d,l-mandélate | 114 |
| 4 | 2-Me | H | d,l-mandélate | 112 |
| 5 | 4-Me | H | d,l-mandélate | 119 |
| 6 | 3-$CF_3$ | H | d,l-mandélate | 126 |
| 7 | 2-OMe | H | chlorhydrate | 170 |
| 8 | 3-OMe | H | d,l-mandélate | 117 |
| 9 | 4-OMe | H | d,l-mandélate | 129 |
| 10 | 2-OEt | H | d,l-mandélate | 135 |
| 11 | 3,4-$(OMe)_2$ | H | d,l-mandélate | 108 |
| 12 | H | Me | d,l-mandélate | 118 |
| 13 | H | $CO_2Et$ | huile | $Eb_{0,4}$ = 149-151 |
| 14 | 3-$CF_3$ | $CH_2CH_2OH$ | chlorhydrate | 102 |
| 15 | 3,4,5-$(OMe)_3$ | $CH(Me)_2$ | citrate | 94-95 |
| 16 | 3,4,5-$(OMe)_3$ | $CO_2Et$ | base | 64 |
| 17 | 3-$CF_3$ | $CH(Me)_2$ | fumarate | 117 |
| 18 | 3-Me | $CO_2Et$ | huile | |
| 19 | 3-$CF_3$ | $CO_2Et$ | huile | |
| 20 | 3,4,5-$(OMe)_3$ | Me | citrate | |
| 21 | 4-Cl | Me | chlorhydrate | 149-151 |
| 22 | 3-$CF_3$ | Me | chlorhydrate | 111-112 |
| 23 | 3,4,5-$(OMe)_3$ | $CH_2$-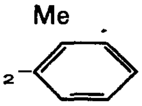 | fumarate | 160-161 |
| 24 | 4-Cl | H | chlorhydrate | 157-159 |
| 25 | 3,4-$Cl_2$ | H | chlorhydrate | 142,5-144 |
| 26 | 3,4-$Cl_2$ | $CH_2$-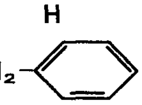 | chlorhydrate | 184-186 |
| 27 | 3,4-$Cl_2$ | Me | chlorhydrate | 119-120 |
| 28 | 4-Cl | $CO_2Et$ | huile | |
| 29 | 4-Cl | $CH_2$-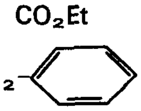 | chlorhydrate | 171,5-173 |
| 30 | 3,4-$Cl_2$ | $CH_2CH_2OH$ | chlorhydrate | 115,5-117 |
| 31 | 4-$O^iPr$ | H | fumarate | 140-141,5 |

*Tableau* (suite)

| Composé | X | R | Sel | P.f. (° C) |
|---|---|---|---|---|
| 32 | 3,4,5-$(OMe)_3$ | 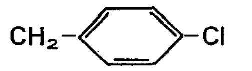 | fumarate | 178-180 |
| 33 | 4-OMe | 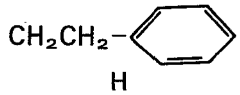 | chlorhydrate | 165-167 |
| 34 | 4-$^{i}$Pr | H | fumarate | 145-146 |
| 35 | 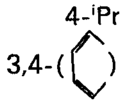 | H | fumarate | 170-171,5 |
| 36 | 4-OMe | 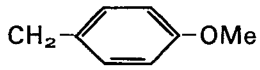 | fumarate | 138-139 |
| 37 | 3,4,5-$(OMe)_3$ | 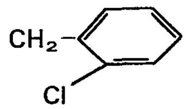 | fumarate | 167,5-168,5 |
| 38 | 4-OMe | 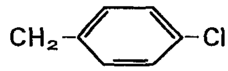 | fumarate | 167-168 |
| 39 | 4-OMe | 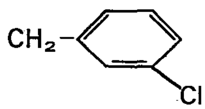 | fumarate | 152-153 |
| 40 | 4-OMe | 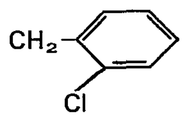 | fumarate | 137-138 |
| 41 | 2,4-$Cl_2$ | $CH_2CH_2OH$ | fumarate | 106-107 |
| 42 | 3,4,5-$(OMe)_3$ | 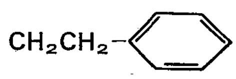 | fumarate | 147-148 |
| 43 | 4-Cl | 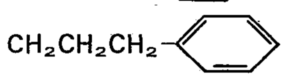 | fumarate | 148-149 |
| 44 | 3,4,5-$(OMe)_3$ | 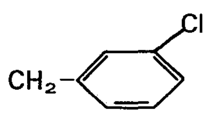 | fumarate | 180-181,5 |
| 45 | 4-OMe | 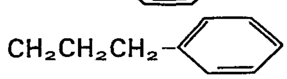 | fumarate | 125,5-127 |
| 46 | 3,4-$Cl_2$ | $CO_2Me$ | huile | |
| 47 | 2,4-$Cl_2$ | $CO_2Et$ | huile | Eb = 164-168° C/0,001 mm |
| 48 | 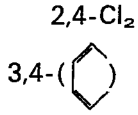 | $CO_2Et$ | huile | Eb = 183-185° C/0,003 mm |
| 49 | 4-Br | H | chlorhydrate | 205-207 |

Les composés de l'invention (I) ont été soumis à des essais pharmacologiques qui ont montré leur activité antidépressive.

La toxicité des composés a été déterminée chez la souris par voie i.p. La $DL_{50}$ varie de 30 à 1000 mg/kg.

L'activité antidépressive a été déterminée selon le test de l'antagonisme vis-à-vis de la ptose réserpinique (Gouret C. *et al.*, «J. Pharmacol.» [Paris, 8, 333-350 (1977)].

Les souris (mâles, Cdl Charles River, France, 18-22 g) reçoivent simultanément les produits à étudier ou le solvant (voie i.p.), et la réserpine (4 mg/kg, voie s.c.).

60 min plus tard, le degré de ptose palpébrale est estimé au moyen d'une échelle de cotation (0 à 4) pour chaque souris.

A chaque dose, la moyenne de cotation et le pourcentage de variation par rapport au lot de contrôle sont calculés.

Pour chaque produit, la $DA_{50}$, ou dose qui diminue de 50% le score moyen de ptose par rapport aux contrôles, est déterminée graphiquement.

La $DA_{50}$ varie de 4 à 10 mg/kg par voie i.p.

L'activité antidépressive a également été déterminée selon le test de potentialisation des hochements de tête («head-twitches») provoqués par le

L-5-hydroxytryptophane. [Van Riezen, H. (1972), «Arch. Int. Pharmacology», *198*, 256-269.]

Le protocole en est le suivant:

24 h avant l'expérience, on place les animaux dans le laboratoire où s'effectuera la manipulation. Le jour de l'expérience, les souris sont pesées et endormies. Puis on injecte les produits à tester par voie i.p. avant l'injection de L5-HTP par voie s.c. à 125 mg/kg en suspension dans du tween. On compte le nombre de «head-twitches» 30 min après l'injection de L5-HTP et pendant 60 s.

Pour chaque dose, on calcule le nombre moyen de «head-twitches» et le pourcentage de variation par rapport aux témoins. La dose active 50 est établie à partir d'une courbe.

La $DA_{50}$ varie de 0,1 à 5 mg/kg par voie i.p.

Les résultats pharmacologiques montrent que les composés de l'invention peuvent être utiles pour le traitement de la dépression.

Les composés de l'invention peuvent être présentés sous toute forme appropriée pour l'administration par voie orale ou parentérale, par exemple sous la forme de comprimés, dragées, gélules, solutions buvables ou injectables, etc., en association avec tout excipient approprié.

La posologie quotidienne peut aller de 5 à 200 mg.

R: BE23 J: 12-7 PTD12-7a 77427

**Revendications** pour les Etats contractants BE, CH, DE, FR, GB, IT, LI, LU, NL, SE

1. Dérivés de pipéridine répondant à la formule (I)

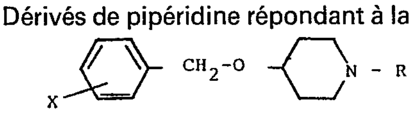 (I)

dans laquelle
R représente soit un atome d'hydrogène, soit un radical $(C_{1-4})$ alkyle,
soit un radical hydroxy $(C_{1-4})$ alkyle,
soit un radical $(C_{1-4})$ alcoxycarbonyle,
soit un radical benzyle pouvant porter un substituant choisi parmi les atomes d'halogène et les radicaux $(C_{1-4})$ alcoxy,
soit le radical phénéthyle,
soit le radical phényl-3 propyle,
X représente un ou plusieurs atomes d'hydrogène ou d'halogène ou radicaux $(C_{1-4})$ alkyle, $(C_{1-4})$ alcoxy, trifluorométhyle, méthylènedioxy ou bien X forme avec le noyau phényle un radical naphtyle,
R et X ne pouvant représenter simultanément des atomes d'hydrogène,
ainsi que leurs sels d'addition aux acides pharmaceutiquement acceptables.

2. Dérivés selon la revendication 1, dans lesquels X représente un ou plusieurs atomes de chlore, ou forme avec le noyau phényle un radical naphtyle ou encore représente trois radicaux méthoxy.

3. Dérivés selon la revendication 2, dans lesquels R est H ou un radical $(C_{1-4})$ alcoxycarbonyle.

4. Procédé de préparation des composés selon la revendication 1, procédé caractérisé en ce que l'on fait réagir un composé (II)

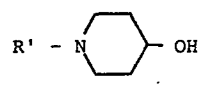

dans lequel
R' représente un radical protecteur de l'azote tel que le radical nitro-4 benzoyle, benzoyle éventuellement substitué, benzyle éventuellement substitué ou alkyle avec un composé (III)

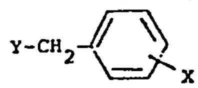

dans lequel Y représente un radical réactif tel qu'un atome de chlore ou de brome; puis soit on élimine le radical protecteur R' s'il ne correspond pas à un radical R, soit on réduit le radical protecteur benzoyle éventuellement substitué, soit on fait réagir un composé (I)

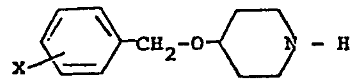

avec un composé RZ dans lequel Z est un groupe réactif de manière à greffer le radical R.

5. Médicament, caractérisé en ce qu'il contient un composé selon l'une des revendications 1 à 3.

6. Composition pharmaceutique, caractérisée en ce qu'elle contient un composé selon l'une des revendications 1 à 3 en association avec tout excipient approprié.

**Revendication** pour l'Etat contractant: AT

Procédé de préparation des dérivés de pipéridine répondant à la formule (I)

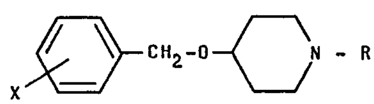 (I)

dans laquelle
R représente soit un atome d'hydrogène, soit un radical $(C_{1-4})$ alkyle,
soit un radical hydroxy $(C_{1-4})$ alkyle,
soit un radical $(C_{1-4})$ alcoxycarbonyle,
soit un radical benzyle pouvant porter un substituant choisi parmi les atomes d'halogène et les radicaux $(C_{1-4})$ alcoxy,
soit le radical phénéthyle,
soit le radical phényl-3 propyle,
X représente un ou plusieurs atomes d'hydrogène ou d'halogène ou radicaux $(C_{1-4})$ alkyle, $(C_{1-4})$ alcoxy, trifluorométhyle, méthylènedioxy ou bien X forme avec le noyau phényle un radical naphtyle,
R et X pouvant représenter simultanément des atomes d'hydrogène,
ainsi que de leurs sels d'addition aux acides pharmaceutiquement acceptables,
procédé caractérisé en ce que l'on fait réagir un composé (II)

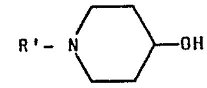

dans lequel
R' représente un radical protecteur de l'azote tel que le radical nitro-4 benzoyle, benzoyle éventuellement substitué, benzyle éventuellement substitué ou alkyle avec un composé (III)

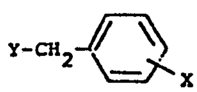

dans lequel Y représente un radical réactif tel qu'un atome de chlore ou de brome; puis on élimine le radical protecteur R' s'il ne correspond pas à un radical R, soit on réduit le radical protecteur benzoyle éventuellement substitué, soit on fait réagir un composé (I)

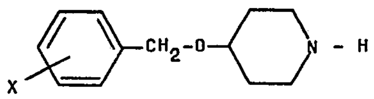

avec un composé RZ dans lequel Z est un groupe réactif de manière à greffer le radical R.

**Patentansprüche** für die Vertragsstaaten BE, CH, DE, FR, GB, IT, LI, LU, NL, SE

1. Piperidinderivate, die der Formel (I)

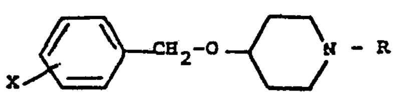 (I)

entsprechen, in welcher
R für ein Wasserstoffatom, einen $(C_{1-4})$-Alkylrest, einen Hydroxy-$(C_{1-4})$-alkylrest, einen $(C_{1-4})$Alkoxycarbonylrest, einen Benzylrest, der einen Substituenten aus der Gruppe Halogenatome und $(C_{1-4})$-Alkoxyreste tragen kann, den Phenäthylrest oder den 3-Phenylpropylrest steht,
X ein oder mehrere Wasserstoff- oder Halogenatome oder $(C_{1-4})$-Alkylreste, $(C_{1-4})$-Alkoxyreste, Trifluormethylreste, Methylendioxyreste bedeutet oder X gemeinsam mit dem Phenylkern einen Naphthylrest bildet, wobei R und X nicht gleichzeitig für Wasserstoffatome stehen können,
sowie deren Säureadditionssalze mit pharmazeutisch verwendbaren Säuren.

2. Derivate nach Anspruch 1, bei welchen X für ein oder mehrere Chloratome steht oder mit dem Phenylkern einen Naphthylrest bildet oder drei Methoxyreste darstellt.

3. Derivate nach Anspruch 2, bei welchen R für H oder einen $(C_{1-4})$-Alkoxycarbonylrest steht.

4. Verfahren zur Herstellung von Verbindungen nach Anspruch 1, dadurch gekennzeichnet, dass man eine Verbindung (II)

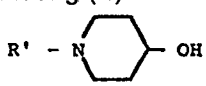

in welcher R' für eine Stickstoffschutzgruppe, wie den 4-Nitrobenzoylrest, einen ggf. substituierten Benzoylrest, einen ggf. substituierten Benzylrest oder Alkyl steht, mit einer Verbindung (III)

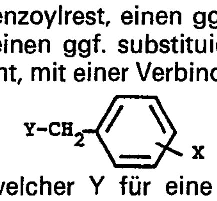

umsetzt, in welcher Y für eine reaktionsfähige Gruppe wie Chlor oder Brom steht; dann eliminiert man entweder die Schutzgruppe R', wenn sie nicht einem Rest R entspricht, oder man reduziert die ggf. substituierte Benzoylschutzgruppe oder man lässt eine Verbindung (I)

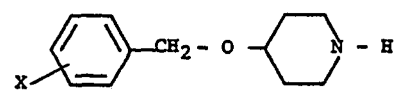

mit einer Verbindung RZ reagieren, in welcher Z für eine reaktionsfähige Gruppe steht, die eine Aufpfropfung des Restes R bewirkt.

5. Arzmeimittel, dadurch gekennzeichnet, dass es eine Verbindung nach irgendeinem der Ansprüche 1 bis 3 enthält.

6. Pharmazeutische Zusammensetzung, dadurch gekennzeichnet, dass sie eine Verbindung nach irgendeinem der Ansprüche 1 bis 3 gemeinsam mit jedem geeigneten Trägermaterial enthält.

**Patentanspruch** für den Vertragsstaat: AT

Verfahren zur Herstellung von Piperidinderivaten der Formel (I)

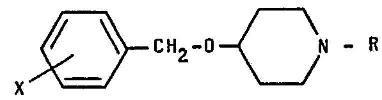 (I)

in welcher
R für ein Wasserstoffatom, einen $(C_{1-4})$-Alkylrest, einen Hydroxy-$(C_{1-4})$-alkylrest, einen $(C_{1-4})$-Alkoxycarbonylrest, einen Benzylrest, der einen Substituenten aus der Gruppe Halogenatome und $(C_{1-4})$-Alkoxyreste tragen kann, den Phenäthylrest oder den 3-Phenylpropylrest steht,
X ein oder mehrere Wasserstoff- oder Halogenatome oder $(C_{1-4})$-Alkylreste, $(C_{1-4})$Alkoxyreste, Trifluormethylreste, Methylendioxyreste bedeutet oder X gemeinsam mit dem Phenylkern einen Naphthylrest bildet, wobei R und X nicht gleichzeitig für Wasserstoffatome stehen können,
sowie deren Säureadditionssalze mit pharmazeutisch verwendbaren Säuren, dadurch gekennzeichnet, dass man eine Verbindung (II)

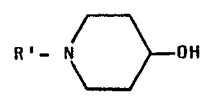

in welcher R' für eine Stickstoffschutzgruppe, wie den 4-Nitrobenzoylrest, einen ggf. substituierten Benzoylrest, einen ggf. substituierten Benzylrest oder Alkyl steht, mit einer Verbindung (III)

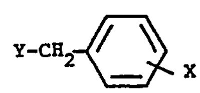

umsetzt, in welcher Y für eine reaktionsfähige Gruppe wie Chlor oder Brom steht; dann eliminiert man entweder die Schutzgruppe R', wenn sie nicht einem Rest R entspricht, oder man reduziert die ggf. substituierte Benzoylschutzgruppe oder man lässt eine Verbindung (I)

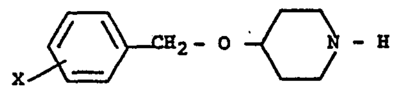

mit einer Verbindung RZ reagieren, in welcher Z für eine reaktionsfähige Gruppe steht, die eine Aufpfropfung des Restes R bewirkt.

**Claims** for the Contracting States BE, CH, DE, FR, GB, IT, LI, LU, NL, SE

1. Piperidine derivatives of Formula (I)

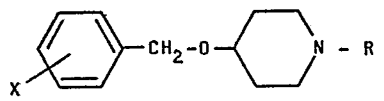

(I)

wherein
R represents either a hydrogen atom, or a $(C_{1-4})$ alkyl radical
or a hydroxy $(C_{1-4})$alkyl radical,
or a $(C_{1-4})$alkoxycarbonyl radical,
or a benzyl radical optionally bearing a substituent selected from halogen atoms and $(C_{1-4})$alkoxy radicals,
or the phenethyl radical
or the 3-phenyl-propyl radical,
X represents one or more hydrogen or halogen atoms or $(C_{1-4})$alkyl, $(C_{1-4})$alkoxy, trifluoromethyl, methylenedioxy radicals, or X together with the phenyl ring forms a naphthyl radical,
provided that R and X do not represent simultaneously hydrogen atoms,
and their pharmaceutically acceptable acid addition salts.

2. Derivatives according to Claim 1, wherein X represents one or more chlorine atoms, or X together with the phenyl ring forms a naphthyl radical, or represents three methoxy radicals.

3. Derivatives according to Claim 2, wherein R ist H or a $(C_{1-4})$alkoxycarbonyl radical.

4. A process for the preparation of compounds according to Claim 1, characterized in that a compound (II)

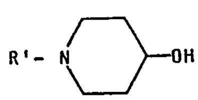

wherein R′ represents a protecting radical for the nitrogen, such as a 4-nitro-benzoyl radical, optionally substituted benzoyl radical, optionally substituted benzyl radical or alkyl radical,
is reacted with a compound (III)

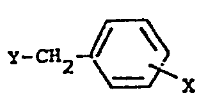

wherein Y represents a reactive radical such as bromine or chlorine; then the protecting radical R′ is either eliminated when it does not correspond to a R radical, or the protecting, optionally substituted, benzoyl group is reduced,
either a compound (I)

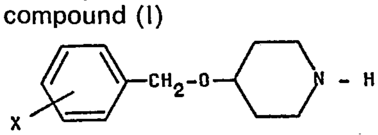

is reacted with a compound RZ, wherein Z is a reactive group, so as to graft the R radical.

5. Medicament, characterized in that it contains a compound according to any one of Claims 1 to 3.

6. Pharmaceutical composition, characterized in that it contains a compound according to any one of Claims 1 to 3 in association with any suitable excipient.

**Claim** for the Contracting State: AT

Process for the preparation of piperidine derivatives of Formula (I)

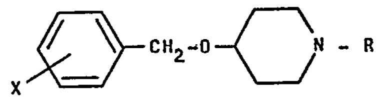

(I)

wherein
R represents either a hydrogen atom, or a $(C_{1-4})$ alkyl radical
or a hydroxy $(C_{1-4})$alkyl radical,
or a $(C_{1-4})$alkoxycarbonyl radical,
or a benzyl radical optionally bearing a substituent selected from halogen atoms and $(C_{1-4})$alkoxy radicals,
or the phenethyl radical
or the 3-phenyl-propyl radical,
X represents one or more hydrogen or halogen atoms or $(C_{1-4})$alkyl, $(C_{1-4})$alkoxy, trifluoromethyl, methylenedioxy radicals, or X together with the phenyl ring forms a naphthyl radical,
provided that R and X do not represent simultaneously hydrogen atoms,
and their pharmaceutically acceptable acid addition salts, process characterized in that a compound (II)

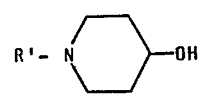

wherein R′ represents a protecting radical for the nitrogen, such as a 4-nitro-benzoyl radical, optionally substituted benzoyl radical, optionally substituted benzyl radical or alkyl radical,
is reacted with a compound (III)

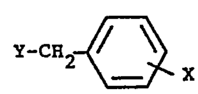

wherein Y represents a reactive radical such as bromine or chlorine; then the protecting radical R′ is either eliminated when it does not correspond to a R radical, or the protecting, optionally substituted, benzoyl group is reduced,
either a compound (I)

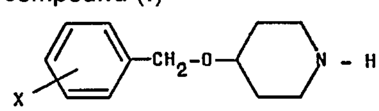

is reacted with a compound RZ, wherein Z is a reactive group, so as to graft the R radical.